# EUROPEAN PATENT SPECIFICATION

(11) **EP 3 772 539 B1**
(45) Date of publication and mention of the grant of the patent: **03.06.2026**
(21) Application number: 19190650.2
(22) Date of filing: 08.08.2019
(51) Int. Cl.: C12N 9/10, A23C 9/13

(54) **SIALYLTRANSFERASES FROM STREPTOCOCCUS BOVIS FOR THE PRODUCTION OF 6'-SIALYLLACTOSE**
SIALYLTRANSFERASEN VON STREPTOCOCCUS BOVIS ZUR HERSTELLUNG VON 6'-SIALYLLACTOSE
SIALYLTRANSFÉRASES DE STREPTOCOCCUS BOVIS POUR LA PRODUCTION DE 6'-SIALYLLACTOSE

(43) Date of publication of application: 10.02.2021
(73) Proprietor: Chr. Hansen HMO GmbH, 53619 Rheinbreitbach (DE)
(72) Inventor: JENNEWEIN, Stefan, 53605 Bad Honnef (DE); WARTENBERG, Dirk, 55435 Gau-Algesheim (DE)

(56) References cited:
- WO-A1-2014/153253
- ZHIJIE LIU ET AL: "Development of Multiplex PCR Assays for the Identification of the 33 Serotypes of Streptococcus suis", PLOS ONE, vol. 8, no. 8, 9 August 2013 (2013-08-09), pages e72070, XP055374000, DOI: 10.1371/journal.pone.0072070
- 5 April 2011 (2011-04-05), XP055669738, Retrieved from the Internet <URL:https://www.uniprot.org/uniprot/E9NQ24.txt>
- NCBI: "Lipooligosaccharide sialyltransferase [Streptococcus suis] - Protein - NCBI", 12 May 2013 (2013-05-12), XP055661366, Retrieved from the Internet <URL:https://www.ncbi.nlm.nih.gov/protein/WP_002936327> [retrieved on 20200123]
- DROUILLARD S ET AL: "Efficient synthesis of 6'-sialyllactose, 6,6'-disialyllactose, and 6'-KDO-lactose by metabolically engineered E. coli expressing a multifunctional sialyltransferase from the Photobacterium sp. JT-ISH-224", CARBOHYDRATE RESEARCH, PERGAMON, GB, vol. 345, no. 10, 2 July 2010 (2010-07-02), pages 1394 - 1399, XP027118796, ISSN: 0008-6215, [retrieved on 20100224]
- SPRENGER GEORG A ET AL: "Production of human milk oligosaccharides by enzymatic and whole-cell microbial biotransformations", JOURNAL OF BIOTECHNOLOGY, ELSEVIER, AMSTERDAM, NL, vol. 258, 29 July 2017 (2017-07-29), pages 79 - 91, XP085189451, ISSN: 0168-1656, DOI: 10.1016/J.JBIOTEC.2017.07.030

## Description

The present invention relates to genetically engineered cells for producing 6'-sialyllactose and to a method for producing 6'-sialyllactose using the genetically engineered cells.

### Background

More than 150 structurally distinct human milk oligosaccharides (HMOs) have been identified to date. Although HMOs represent only a minor amount of total human milk nutrients, their beneficial effects on the development of breast-fed infants became evident over the past decades.

Among the HMOs, sialylated HMOs (SHMOs) were observed to support the resistance of infants to enteropathogenic bacteria and viruses. Interestingly, recent studies further demonstrated a protective effect of long-chained SHMO against necrotizing enterocolitis, which is one of the most common and lethal diseases in preterm infants. In addition, SHMOs are believed to support an infant's brain development and its cognitive capabilities. Also, sialylated oligosaccharides have been shown to neutralize enterotoxins of various pathogenic microbes including *Escherichia coli, Vibrio cholerae* and *Salmonella.* Further, it was found that sialylated oligosaccharides interfere with the colonization of the gut by *Helicobacter pylori* and can thereby prevent or inhibit gastric and duodenal ulcers.

Among the sialylated oligosaccharides in human milk, 3'-sialyllactose, 6'-sialyllactose, sialyllacto-*N*-tetraose a, sialyllacto-*N*-tetraose b, sialyllacto-*N*-tetraose c and disialyllacto-*N*-tetraose are the most prevalent members.

Since sialylated oligosaccharides have a complex structure, their chemical or (chemo-)enzymatic syntheses are challenging and associated with extensive difficulties, e.g. control of stereochemistry, formation of specific linkages, availability of feedstocks, etc. As a consequence, commercially available sialylated oligosaccharides have been very expensive due to their low quantity in natural sources.

To overcome lack of commercial availability of SHMOs, efforts in metabolic engineering of microorganisms to produce sialylated oligosaccharides have been made, since this approach appears to be the most promising way for producing HMOs in an industrial scale.

Microbial fermentation uses genetically modified microorganisms which (over-) express at least one heterologous glycosyltransferase. Upon cultivation of such microorganisms in a medium and under conditions permissive for the microorganisms to express said heterologous glycosyltransferase, an HMO can be produced by said microorganisms and recovered from the culture medium or cell lysate.

However, glycosyltransferases, including sialyltransferases, typically use a broad spectrum of substrate such that their (over-)expression for the production of a desired oligosaccharide usually leads to undesired by-products. Typically, these by-products are oligosaccharides too, but have to be removed from the desired oligosaccharide preparation for the product's commercial use. However, removing such by-products from the desired oligosaccharide might be difficult and cumbersome.

Several sialyltransferases (SiaTs) from bacterial species have been identified and characterized to date, e. g. from *Neisseria, Campylobacter, Pasteurella, Helicobacter* and *Photobacterium,* as well as from mammals and viruses. Sialyltransferases have been generally classified into six glycosyltransferase (GT) families, based on protein sequence similarities. All eukaryotic and viral sialyltransferases are grouped into the GT family 29, whereas bacterial SiaTs are grouped into one the GT4, GT38, GT42, GT52 or GT80 family. Besides, sialyltransferases and polysialyltransferases can be distinguished due to the glycosidic linkages that they form, e. g. into α-2,3-, α-2,6- and α-2,8-sialyltransferases. All these sialyltransferases transfer the sialic acid residue from cytidine 5'-monophosphate sialic acid (e. g. CMP-NeuNAc) to a variety of acceptor molecules, usually a galactose (Gal) moiety, an *N*-acetylgalactosamine (GalNAc) moiety or an *N*-acetylglucosamine (GlcNAc) moietiy or another sialic acid (Sia) moiety.

The amino acid sequences of Lipooligosaccharide sialyltransferases from *Streptococcus suis* as represented by SEQ ID No. 1 and SEQ ID No. 2 are present e.g. in the Uniprot database under accession number E9NQ24, and the NCBI database under accession number WP_002936327. In addition, Liu et al. (PLOS ONE 8 (2013) e72070) refers to database entries disclosing SEQ ID No. 1 and SEQ ID No. 2.

Several bacterial α-2,6-sialyltransferases were well characterized in the past and are already proven to be suitable for the production of 6'-sialyllactose (6'-SL). The most commonly used enzymes for microbial 6'-SL production originate from marine bacteria: Pst-6 of *Photobacterium sp.* JT-ISH-224, St0160 of *Photobacterium damselae* JT0160, PlsT6 of *Photobacterium leiognathi* JT-SHIZ-119 and PlsT6 of *Photobacterium leiognathi* JT-SHIZ-145. However, these known bacterial α-2,6-sialyltransferases also produce disialyllactose as undesired by-product.

Drouillard et al. (Carbohydrate Research 345 (2010) 1394-1399) discloses the production of 6'-sialyllactose by an engineered *E. coli* cell which encodes for the sialyltransferase gene of the marine bacterium *Photobacterium sp.* JT-ISH-224. Due to the promiscuity of this enzyme (broad acceptor/donor substrate acceptance) by-products such as 6,6'-disialyllactose and KDO-lactose are formed during cultivation. Only by downregulating the expression of the sialyltransferase gene, the accumulation of these carbohydrate by-products can be restricted. However, downregulation of sialyltransferase gene expression is an undesired compromise since this limits the productivity of the production process as well as its industrial applicability.

Mehr and Withers (Glycobiology 26 (2016): 353-359) describe sialidase activity (de-sialylation/hydrolysis of a sialylated carbohydrate) and trans-sialidase activity (transfer of a sialic acid residue from a sialylated carbohydrate to an acceptor substrate) of bacterial sialyltransferases from the glycosyltransferase family 80 using the representative α-2,6-sialyltransferases St0160 of *Photobacterium damselae* JT0160 and Pst-6 of *Photobacterium sp.* JT-ISH-224. Considering the sialyltransferases' desired application in processes for the production of a distinct sialylated product such as 6'-sialyllactose, enzymatic activities leading to product conversion and/or degradation are obviously counterproductive.

International Publication WO 2014/153253 A1 discloses a method for engineering bacteria to produce sialylated oligosaccharides such as 6'-SL, and the use thereof in the prevention of infections.

Sprenger et al. (Journal of Biotechnology 258 (2017) 79-91) reviews methods for producing human milk oligosaccharides including 6'-SL.

Prior art clearly discloses major limitations for the economic production of sialylated oligosaccharides such as 6'-sialyllactose in industrial scale. In particular, prior art does not teach how to prevent formation of disialyllactose by a 6'-sialyllactose producing bacterial strain in a feasible manner.

It was therefore an object of the present invention to provide an improved method for the production of 6'-sialyllactose by microbial fermentation in an industrial scale, wherein concomitant formation of undesired by-products such as disialyllactose is prevented or at least significantly reduced.

### Summary

The object is solved by the identification of α-2,6-sialyltransferases which exhibit no specificity for a sialyllactose as a substrate for sialylation reactions catalyzed by the enzyme, and the provision of their use in the production of 6'-sialyllactose.

The invention is set out in the appended set of claims.

### Brief description of the drawings

- Fig. 1: shows the structural formula representing 6'-sialyllactose.
- Fig. 2: shows the result of a thin layer chromatography indicating the carbohydrates detectable after a cultivation of an engineered *E*. *coli* strain (as described in example 2) encoding the sialyltransferase *plsT6* (F). S: standard substance.
- Fig. 3: displays the results of a thin layer chromatography from an exemplary in vitro assay illustrating sialyltransferase activity of various enzymes when providing CMP-NeuNAc as donor substrate and 20 mM lactose as acceptor substrate.
- Fig. 4: displays the results of a thin layer chromatography from an exemplary in vitro assay illustrating sialyltransferase activity of various enzymes when providing CMP-NeuNAc as donor substrate and 20 mM 6'-SL as acceptor substrate.
- Fig. 5: displays the results of a thin layer chromatography from an exemplary in vitro assay illustrating sialyltransferase activity of a sialyltransferase from *S. suis* when providing CMP-NeuNAc as donor substrate and 20 mM lactose or 20 mM 6'-SL as acceptor substrate.

### Detailed description

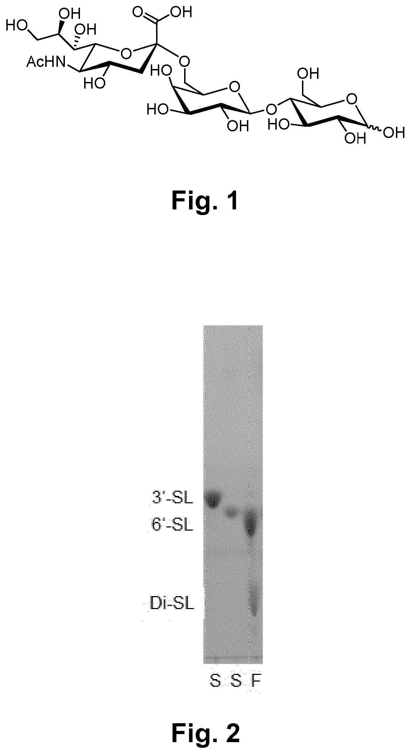

According to a first aspect, provided is a genetically engineered cell for the production of 6'-sialyllactose in the cell. In various embodiments, the cell has been genetically engineered to possess said lactose-accepting α-2,6-sialyltransferase. The term "6'-sialyllactose" (6'-SL) as used herein refers to a trisaccharide, wherein said trisaccharide consists of the monosaccharide residues glucose (Glc), galactose (Gal) and N-acetylneuraminic acid/sialic acid (NeuNAc) to form a compound possessing the chemical structure NeuNAc(α2,6)Gal(β1,4)Glc as shown in Fig. 1.

The term "sialyltransferase" as used herein refers to polypeptides being able to possess sialyltransferase activity. "Sialyltransferase activity" refers to the transfer of an N-acetylneuraminic acid (NeuNAc) residue from a donor substrate, typically CMP-NeuNAc, to an acceptor molecule. The term "sialyltransferase" comprises functional fragments of the sialyltransferases described herein, functional variants of the sialyltransferases described herein, and functional fragments of the functional variants. "Functional" in this regard means that the fragments and/or variants possess sialyltransferase activity. Functional fragments of a sialyltransferase encompass truncated versions of a sialyltransferase as encoded by its naturally occurring gene, which truncated version is capable of possessing sialyltransferase activity. Examples of truncated versions are sialyltransferases which do not comprise a so-called leader sequence which typically directs the polypeptide to a specific subcellular localization. Typically, such leader sequence is removed from the polypeptide during its subcellular transport, and is absent in the naturally occurring mature sialyltransferase.

The α-2,6-sialyltransferase of the present disclosure is capable of transferring a *N-*acetylneuraminic acid residue from a donor substrate to an acceptor molecule. The term "capable of" with respect to the heterologous sialyltransferase refers to the sialyltransferase activity of the heterologous sialyltransferase and the provision that suitable reaction conditions are required for the heterologous sialyltransferase to possess its enzymatic activity. In the absence of suitable reaction conditions, the heterologous sialyltransferase does not possess its enzymatic activity, but retains its enzymatic activity and possesses its enzymatic activity when suitable reaction conditions are restored. Suitable reaction conditions include the presence of a suitable donor substrate, the presence of suitable acceptor molecules, the presence of essential cofactors such as - for example - monovalent or divalent ions, a pH value in an appropriate range, a suitable temperature and the like. It is not necessary that the optimum values for each and every factor effecting the enzymatic reaction of the heterologous sialyltransferase is met, but the reaction conditions have to be such that the heterologous sialyltransferase performs its enzymatic activity. Accordingly, the term "capable of" excludes any conditions upon which the enzymatic activity of the heterologous sialyltransferase has been irreversibly impaired, and also excluded exposure of the heterologous sialyltransferase to any such condition. Instead, "capable of" means that the sialyltransferase is enzymatically active, i.e. possesses its sialyltransferase activity, if permissive reactions conditions (where all requirements being necessary for the sialyltransferase to perform its enzymatic activity) are provided to the sialyltransferase.

Sialyltransferases can be distinguished on the type of glycosidic linkage they form. As used herein, the terms "α-2,3-sialyltransferase" and "α-2,3-sialyltransferase activity" refer to polypeptides and their enzymatic activity which add sialic acid with an alpha-2,3 linkage to galactose or a galactose residue of the acceptor molecule. Likewise, the terms "α-2,6-sialyltransferase" and "α-2,6-sialyltransferase activity" refer to polypeptides and their enzymatic activity which add sialic acid with an alpha-2,6 linkage to galactose or a galactose residue of the acceptor molecule.

The α-2,6-sialyltransferase of the present disclosure is a lactose-accepting sialyltransferase. Hence, the disaccharide lactose is an acceptor substrate of the α-2,6-sialyltransferase for the transfer of a NeuNAc moiety from the donor substrate CMP-NeuNAc.

The α-2,6-sialyltransferase of the present disclosure does not accept a sialyllactose, i.e. 3'-SL or 6'-SL, as substrate. More specifically, the α-2,6-sialyltransferase of the present disclosure neither accepts a sialyllactose as an acceptor substrate for the transfer of a NeuNAc moiety from CMP-NeuNAc as donor substrate nor as a donor substrate for the transfer of the NeuNAc moiety from said sialyllactose to lactose as acceptor substrate as determined by *in vitro* sialylation reactions as described herein below in example 3. Hence, the α-2,6-sialyltransferase of the present disclosure is not able to catalyze formation of disialyllactose. Furthermore, the α-2,6-sialyltransferase of the present disclosure does not possess trans-sialidase activity.

In an embodiment, the α-2,6-sialyltransferase comprises an amino acid sequence that is at least 98 %, 99 %, 99.1 %, 99.2 %, 99.3 %, 99.4 %, 99.5 %, 99.6 %, 99.7 %, 99.8 %, 99.9 %, or 100 % identical to one of the amino acid sequences as represented by SEQ ID No. 1 and SEQ ID No. 2.

In some embodiments, the α-2,6-sialyltransferase comprises an amino acid sequence as represented by SEQ ID No. 1 or SEQ ID No. 2.

The amino acid sequences as represented by SEQ ID No. 1 and SEQ ID No. 2 were retrieved from genomic databases that were searched for putative sialyltransferases. The amino acid sequences as represented by SEQ ID No. 1 and SEQ ID No. 2 were found to be encoded by nucleotide sequences that are present in the genome of the bacterial cells of the genus *Streptococcus suis.* Although said nucleotide sequences were not annotated at the time of their retrieval from the databases, they were annotated as putative sialyltransferases in the meantime. However, annotation as putative sialyltransferase occurred without identification or classification of their enzymatic activities.

The term "genetically engineered" as used herein refers to the modification of the cell's genetic make-up using molecular biological methods. The modification of the cell's genetic make-up may include the transfer of genes within and/or across species boundaries, inserting, deleting, replacing and/or modifying nucleotides, triplets, genes, open reading frames, promoters, enhancers, terminators and other nucleotide sequences mediating and/or controlling gene expression. The modification of the cell's genetic make-up aims to generate a genetically modified organism possessing particular, desired properties. Genetically engineered cells can contain one or more genes that are not present in the native (not genetically engineered) form of the cell. Techniques for introducing exogenous nucleic acid molecules and/or inserting exogenous nucleic acid molecules (recombinant, heterologous) into a cell's hereditary information for inserting, deleting or altering the nucleotide sequence of a cell's genetic information are known to the skilled artisan. Genetically engineered cells can contain one or more genes that are present in the native form of the cell, wherein said genes are modified and reintroduced into the cell by artificial means. The term "genetically engineered" also encompasses cells that contain a nucleic acid molecule being endogenous to the cell, and that has been modified without removing the nucleic acid molecule from the cell. Such modifications include those obtained by gene replacement, site-specific mutations, and related techniques.

In some embodiments, the lactose-accepting α-2,6-sialyltransferase is a heterologous α-2,6-sialyltransferase.

The term "heterologous" as used herein refers to a polypeptide, amino acid sequence, nucleic acid molecule or nucleotide sequence that is foreign to a cell or organism, i.e. to a polypeptide, amino acid sequence, nucleic acid molecule or nucleotide sequence that does not naturally occurs is said cell or organism. A "heterologous sequence" or a "heterologous nucleic acid" or "heterologous polypeptide", as used herein, is one that originates from a source foreign to the particular host cell (e.g. from a different species), or, if from the same source, is modified from its original form. Thus, a heterologous nucleic acid operably linked to a promoter is from a source different from that from which the promoter was derived, or, if from the same source, is modified from its original form. The heterologous sequence may be stably introduced, e.g. by transfection, transformation, conjugation or transduction, into the genome of the host microbial host cell, thus representing a genetically modified host cell. Techniques may be applied which will depend on the host cell the sequence is to be introduced. Various techniques are known to a person skilled in the art and are, e.g., disclosed in Sambrook et al., Molecular Cloning: A Laboratory Manual, 2nd Ed., Cold Spring Harbor Laboratory Press, Cold Spring Harbor, N.Y. (1989). Accordingly, a "heterologous polypeptide" is a polypeptide that does not naturally occur in the cell, and a "heterologous sialyltransferase" is a sialyltransferase that does not naturally occur in the cell.

The genetically engineered cell is a prokaryotic cell or a eukaryotic cell. Preferably, the genetically engineered cell is a microbial cell. Appropriate microbial cells include yeast cells, bacterial cells, archaebacterial cells, algae cells, and fungal cells.

In an additional and/or alternative embodiment, the microbial cell is a prokaryotic cell, preferably a bacterial cell, more preferably a bacterial cell selected from the group consisting of *Bacillus, Lactobacillus, Lactococcus, Enterococcus, Bifidobacterium, Sporolactobacillus spp., Micromomospora spp., Micrococcus spp., Rhodococcus spp.,* and *Pseudomonas.* Suitable bacterial species are *Bacillus subtilis, Bacillus licheniformis, Bacillus coagulans, Bacillus thermophilus, Bacillus laterosporus, Bacillus megaterium, Bacillus mycoides, Bacillus pumilus, Bacillus lentus, Bacillus cereus, Bacillus circulans, Bifidobacterium longum, Bifidobacterium infantis, Bifidobacterium bifidum, Citrobacter freundii, Clostridium cellulolyticum, Clostridium Ijungdahlii, Clostridium autoethanogenum, Clostridium acetobutylicum, Corynebacterium glutamicum, Enterococcus faecium, Enterococcus thermophiles, Escherichia coli, Erwinia herbicola (Pantoea agglomerans), Lactobacillus acidophilus, Lactobacillus salivarius, Lactobacillus plantarum, Lactobacillus helveticus, Lactobacillus delbrueckii, Lactobacillus rhamnosus, Lactobacillus bulgaricus, Lactobacillus crispatus, Lactobacillus gasseri, Lactobacillus casei, Lactobacillus reuteri, Lactobacillus jensenii, Lactococcus lactis, Pantoea citrea, Pectobacterium carotovorum, Proprionibacterium freudenreichii, Pseudomonas fluorescens, Pseudomonas aeruginosa, Streptococcus thermophiles* and *Xanthomonas campestris.*

In an alternative embodiment, the eukaryotic cell is a yeast cell, an insect cell, a plant cell or a mammalian cell. The yeast cell is preferably selected from the group consisting of *Saccharomyces sp.,* in particular *Saccharomyces cerevisiae, Saccharomycopsis sp., Pichia sp.,* in particular *Pichia pastoris, Hansenula sp., Kluyveromyces sp., Yarrowia sp., Rhodotorula sp.,* and *Schizosaccharomyces sp.*

In an additional and/or alternative embodiment, the genetically engineered cell has been transformed to contain and express a nucleic acid molecule which comprises a nucleotide sequence which encodes lactose-accepting α-2,6-sialyltransferase which comprises an amino acid sequence that is at least 98 % identical to one of the amino acid sequences as represented by SEQ ID No. 1 and SEQ ID No.2, wherein the the α-2,6-sialyltransferase is not able to catalyze formation of disialyllactose.

In some embodiments, the nucleotide sequence is selected from the group consisting of nucleotide sequences encoding a polypeptide as represented by any one of SEQ ID No's: 1 and 2.

In the genetically engineered cell, the nucleotide sequence that encodes the lactose-accepting α-2,6-sialyltransferase is operably linked to expression control sequences effecting transcription and/or translation of said nucleotide sequence encoding the lactose-accepting α-2,6-sialyltransferase in the genetically engineered cell.

The term "operably linked" as used herein, refers to a functional linkage between the nucleotide sequence encoding the lactose-accepting α-2,6-sialyltransferase and a second nucleotide sequence, the nucleic acid expression control sequence (such as promoter, operator, enhancer, regulator, array of transcription factor binding sites, transcriptional terminator, ribosome binding site), wherein the expression control sequence affects transcription and/or translation of the nucleic acid corresponding to the nucleotide sequence encoding the lactose-accepting α-2,6-sialyltransferase. Accordingly, the term "promoter" designates DNA sequences which usually "precede" a gene in a DNA polymer and provide a site for initiation of the transcription into mRNA. "Regulator" DNA sequences, also usually "upstream" of (i.e., preceding) a gene in a given DNA polymer, bind proteins that determine the frequency (or rate) of transcriptional initiation. Collectively referred to as "promoter/ regulator" or "control" DNA sequence, these sequences which precede a selected gene (or series of genes) in a functional DNA polymer cooperate to determine whether the transcription (and eventual expression) of a gene will occur. DNA sequences which "follow" a gene in a DNA polymer and provide a signal for termination of the transcription into mRNA are referred to as transcription "terminator" sequences.

In some embodiments, the genetically engineered cell possesses an increased production of one or more nucleotide-activated sugars selected from the group consisting of CMP-N-acetylneuraminic acid, UDP-N-acetylglucosamine, UDP-galactose and GDP-fucose. Preferably, the at least one genetically engineered cell has been genetically engineered to possess an increased production of one or more of said nucleotide-activated sugars. The production of the at least one of said nucleotide activated sugars is increased in the genetically engineered cell as compared to the production of the same nucleotide-activated sugar(s) in the cell prior to be genetically engineered to possess an increased production of at least one of said nucleotide-activated sugars.

In an additional and/or alternative embodiment, the cell has been genetically engineered to overexpress, as compared to the cell prior to be genetically engineered, one or more genes encoding for a polypeptide being capable of possessing an enzymatic activity selected from the group consisting of L-glutamine:D-fructose-6-phosphate aminotransferase, *N*-acetylglucosamine-1-phosphate uridyltransferase, glucosamine-1-phosphate acetyl transferase, phosphor-glucosamine mutase, glucosamine-6-phosphate-*N*-acetyltransferase, *N*-acetylglucosamine-2-epimerase, UDP-*N*-acetylglucosamine-2-epimerase, sialic acid synthase, phosphoenolpyruvate synthase, CMP-sialic acid synthase, UDP-galactose-4-epimerase, galactose-1-phosphate uridylyltransferase, phosphogluco-mutase, glucose-1-phosphate uridylyltransferase, phosphomannomutase, mannose-1-phosphate guanosyltransferase, GDP-mannose-4,6-dehydratase, GDP-L-fucose synthase and fucosekinase/L-fucose-1-phosphate-guanyltransferase.

Overexpression of one or more of said genes increases the amount of the corresponding enzyme(s) in the genetically engineered cell, and hence increases the corresponding enzymatic activity in the cell to enhance intracellular production of at least one of said nucleotide-activated sugars.

Preferably, said nucleotide sequences are operably linked to at least one nucleic acid expression control sequence effecting transcription and/or translation of said nucleotide sequences in the genetically engineered cell.

In an additional and/or alternative embodiment, the genetically engineered cell possesses a glutamine:fructose-6-phosphate aminotransferase (GlmS), preferably a heterologous glutamine:fructose-6-phosphate aminotransferase, more preferably a glutamine:fructose-6-phosphate aminotransferase which is derived from *E. coli* (acc. no. NP_418185), or a functional variant of the *E*. *coli* GlmS. Most preferably, the functional variant is a version of the *E*. *coli* GlmS which shows significantly reduced sensitivity to glucosamine-6-phosphate inhibition as the wild-type enzyme does, as example as encoded by the mutant *glmS* gene (Deng et al., Metab Eng. 7 (2005): 201-214.).

The enzyme glutamine:fructose-6-phosphate aminotransferase (EC 2.6.1.16) catalyzes the conversion of fructose-6-phosphate to glucosamine-6-phosphate using glutamine. This enzymatic reaction is typically considered to be the first step in the hexosamine biosynthesis pathway. Alternative names of the glutamine:fructose-6-phosphate aminotransferase are D-fructose-6-phosphate amidotransferase, GFAT, glucosamine-6-phosphate synthase, hexosephosphate aminotransferase, and L-glutamine-D-fructose-6-phosphate amidotransferase.

In an additional and/or alternative embodiment, the genetically engineered cell comprises a glucosamine-6-phosphate *N*-acetyltransferase (Gna1), preferably a heterologous glucosamine-6-phosphate *N*-acetyltransferase, more preferably a glucosamine-6-phosphate *N*-acetyltransferase which is derived from *S. cerevisiae* (acc. no. NP_116637), or a functional variant of the *S. cerevisiae* Gna1. However, glucosamine-6-phosphate *N*-acetyltransferases, their deduced amino acid sequences and the nucleotides sequences encoding these glucosa mine-6-phosphate *N*-acetyltransferases are known from a variety of different species, and may also be used as suitable glucosamine-6-phosphate *N*-acetyltransferases.

In an additional and/or alternative embodiment, the genetically engineered cell expresses a *N*-acetylglucosamine-6-phosphate phosphatase, preferably a sugar phosphatase of the HAD-like superfamily which catalyzes the conversion of *N-*acetylglucosamine-6-phosphate (GlcNAc6P) to *N*-acetylglucosamine (GlcNAc). The HAD-like superfamily is named after the bacterial enzyme haloacid dehydrogenase and includes phosphatases. A suitable phosphatase of the HAD-like superfamily catalyzing the conversion of GlcNAc6P to GlcNAc may be selected from the group consisting of fructose-1-phosphate phosphatase (YqaB, acc. no. NP_417175) and alpha-D-glucose 1-phosphate phosphatase (YihX, acc. no. NP_418321). The *E. coli* YqaB and *E. coli* YihX enzymes are considered to also act on GlcNAc6P (Lee, S.-W. and Oh, M.-K. (2015) Metabolic Engineering 28: 143-150). In an embodiment, the sugar phosphatase of the HAD-like superfamily catalyzing the conversion of GlcNAc-6-phosphate to GlcNAc is a heterologous enzyme in the genetically engineered cell. In an additional and/or alternative embodiment, the sugar phosphatase of the HAD-like superfamily catalyzing the conversion of GlcNAc6P to GlcNAc is selected from the group consisting of *E. coli* YqaB, *E. coli* YihX, and functional variants thereof.

In an additional and/or alternative embodiment, the genetically engineered cell contains a nucleic acid molecule which comprises a nucleotide sequence encoding a sugar phosphatase of the HAD-like superfamily catalyzing the conversion of GlcNAc6P to GlcNAc. In an additional and/or alternative embodiment, the nucleotide sequence encoding the sugar phosphatase of the HAD-like superfamily catalyzing the conversion of GlcNAc6P to GlcNAc is a heterologous nucleotide sequence. In an additional and/or alternative embodiment, the nucleotide sequence encoding the sugar phosphatase of the HAD-like superfamily catalyzing the conversion of GlcNAc6P to GlcNAc encodes the *E. coli* fructose-1-phosphate phosphatase or the *E. coli* alpha-D-glucose 1-phosphate phosphatase or a functional fragment of one of these two enzymes.

In an additional and/or alternative embodiment, the non-naturally-occurring cell has been genetically engineered to contain a nucleic acid molecule comprising a nucleotide sequence encoding a sugar phosphatase of the HAD-like superfamily catalyzing the conversion of GlcNAc6P to GlcNAc or a functional fragment of said HAD phosphatase and/or to comprise a sugar phosphatase of the HAD-like superfamily catalyzing the conversion of GlcNAc6P to GlcNAc or functional variant thereof.

Nucleotide sequences encoding a suitable sugar phosphatase of the HAD-like superfamily catalyzing the conversion of GlcNAc6P to GlcNAc may be selected from the group of nucleotide sequences encoding *E. coli* YqaB, *E. coli* YihX and functional variants thereof.

In an additional and/or alternative embodiment, the genetically engineered cell possesses a *N*-acetylglucosamine 2-epimerase, preferably the heterologous *N-*acetylglucosamine 2-epimerase Slr1975 of *Synechocystis* sp. PCC 6803 (acc. no. BAL35720).

*N*-acetylglucosamine 2-epimerase (EC 5.1.3.8) is an enzyme that catalyzes the conversion of *N*-acetylglucosamine (GlcNAc) to *N*-acetylmannosamine (ManNAc). The enzyme is a racemase acting on carbohydrates and their derivatives. The systematic name of this enzyme class is *N*-acyl-D-glucosamine 2-epimerase. This enzyme participates in amino-sugar metabolism and nucleotide-sugar metabolism.

In an additional and/or alternative embodiment, the genetically engineered cell comprises a sialic acid synthase or a functional variant thereof, preferably the heterologous sialic acid synthase NeuB of *Campylobacter jejuni* (acc. no. AF305571).

Sialic acid synthase or *N*-acetylneuraminate synthase or *N*-acetylneuraminic acid synthase (EC 2.5.1.56) is an enzyme that catalyzes the conversion of *N*-acetylmannosamine to *N*-acetylneuraminic acid using phosphoenolpyruvate (PEP). The *N*-acetylneuraminic acid synthase (NeuB) is encoded by the *neu*B gene.

In an additional and/or alternative embodiment, the genetically engineered cell synthesizes more PEP than the wildtype of the cell. In an additional and/or alternative embodiment, the genetically engineered cell has been genetically engineered to possess an enhanced PEP biosynthesis pathway. Preferably, the genetically engineered cell has been genetically engineered to possess an increased phosphoenolpyruvate synthase activity, for example in that the *pps*A gene encoding the phosphoenolpyruvate synthase (acc. no. ACT43527) is overexpressed and/or in that the genetically engineered cell contains at least one additional copy of a nucleotide sequence allowing the expression of a phosphoenolpyruvate synthase or a functional variant thereof. Overexpression of *pps*A enhances intracellular PEP synthesis such that more PEP is available for the production of sialic acid. For example, a suitable phosphoenolpyruvate synthase is PpsA of *E*. *coli.*

In an additional and/or alternative embodiment, the genetically engineered cell comprises a CMP-sialic acid synthetase or a functional variant thereof, preferably the heterologous CMP-sialic acid synthetase NeuA of *Campylobacter jejuni* (acc. no. AF305571).

Said CMP-sialic acid synthetase being overexpressed in the cell is capable of transferring a CMP residue onto sialic acid for generating CMP-activated sialic acid (CMP-Neu5Ac).

In an additional and/or alternative embodiment, the at least one genetically engineered cell lacks or possesses a decreased activity of one or more enzymatic activities selected from the group consisting of β-galactosidase activity, glucosamine-6-phosphate deaminase, *N*-acetylglucosamine-6-phosphate deacetylase, *N*-acetylmannosamine kinase, *N*-acetylmannosamine-6-phosphate epimerase and *N*-acetylneuraminic acid aldolase as compared to the cell prior to be genetically engineered.

In an additional and/or alternative embodiment, one or more of the genes encoding a β-galactosidase, a glucosamine-6-phosphate deaminase, a *N*-acetylglucosamine-6-phosphate deacetylase, a *N*-acetylmannosamine kinase, a *N*-acetylmanno samine-6-phosphate epimerase and a *N*-acetylneuraminic acid aldolase has/have been deleted from the genome of the genetically engineered cell or the expression of one or more of the genes encoding a β-galactosidase, a glucosamine-6-phosphate deaminase, a *N*-acetylglucosamine-6-phosphate deacetylase, a *N*-acetylmannosamine kinase, a *N*-acetylmannosamine-6-phosphate epimerase and a *N*-acetylneuraminic acid aldolase has/have been inactivated in the genetically engineered cell.

In an additional and/or alternative embodiment, the at least one genetically engineered cell comprises at least one polypeptide selected from the group consisting of a functional lactose permease, a functional fucose permease and a functional sialic acid transporter (importer), preferably comprises and expresses at least one nucleotide sequence encoding one selected from the group consisting of a functional lactose permease, a functional fucose permease and a functional sialic acid transporter (importer).

In an additional and/or alternative embodiment, the genetically engineered cell possesses activity of at least one glycosyltransferase selected from the group consisting of a β-1,3-*N*-acetylglucosaminyltransferase, a β-1,3-galactosyltrans-ferase, a β-1,4-galactosyltransferase, a α-2,3-sialyltransferase and a α-2,6-sialyltransferase.

Also provided is a method for the production of 6'-sialyllactose in a cell, wherein the method comprises
- providing a genetically engineered cell for the production of 6'-sialyllactose, wherein said cell has been genetically engineered to possess a lactose-accepting α-2,6-sialyltransferase which comprises an amino acid sequence that is at least to 98 % identical to one of the amino acid sequences as represented by SEQ ID No. 1 and SEQ ID No. 2, wherein the α-2,6-sialyltransferase is not able to catalyze formation of disialyllactose;
- cultivating said cell in a culture medium and under conditions permissive for the production of 6'-sialyllactose; and
- optionally recovering said 6'-sialyllactose.

For the production of 6'-sialyllactose, the at least one genetically engineered cell is cultivated in a culture medium and under conditions that is/are permissive for the production of 6'-sialyllactose in said cell.

The culture medium contains at least one carbon source for the genetically engineered cells. The at least one carbon source is preferably selected from the group consisting of glucose, fructose, sucrose, glycerol, and combinations thereof.

In an additional and/or alternative embodiment, the culture medium contains at least one selected from the group consisting of galactose, lactose and sialic acid.

The method comprises the step of recovering 6'-sialyllactose that has been produced in/by the at least one genetically engineered cell during its cultivation in the culture medium. The 6'-sialyllactose can be recovered from the fermentation broth after the genetically engineered cell(s) has/have been removed, for example by centrifugation, and/or can be recovered from the cells, for example in that the cells are harvested from the fermentation broth by centrifugation, and are subjected to a cell lysis step. Subsequently, 6'-SL can be further purified from culture medium and/or cell lysates by suitable techniques known to the skilled artisan. Suitable techniques include microfiltration, ultrafiltration, diafiltration, simulated moving bed type chromatograph, electrodialysis, reverse osmosis, gel filtration, anion exchange chromatography, cation exchange chromatography, and the like.

The present invention will be described with respect to particular embodiments and with reference to drawings, but the invention is not limited thereto but only by the claims. Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination. Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description and drawings provided herein, numerous specific details are set forth. However, it is understood that embodiments of the invention may be practiced without these specific details. In other instances, well-known methods, structures and techniques have not been shown in detail in order not to obscure an understanding of this description.

### Example '1: Identification of α-2,6-sialyltransferases

Gene sequences of characterized or putative sialyltransferases were received from the literature and public databases. In addition, sequence similarity searches were conducted by using the amino acid sequences of known sialyltransferases as template sequences. Protein coding nucleotide sequences were synthesized by GenScript cooperation either, as annotated, in a full-length form or, when a signal peptide is predicted, as a truncated variant lacking the N-terminal signal peptide.

Proper software tools enabling the identification of putative signal peptides are known to the skilled artisan.

The sialyltransferases were either subcloned as an operon with *neuA* into pDEST14 by SLIC using gene specific primers, yielding plasmids of the general kind: pDEST14-*siaT-neuA,* or were directly subcloned by GenScript cooperation into plasmid pET11a using restriction sites *Ndel* and *Bam*HI. Both expression systems allow the IPTG-inducible gene expression. For *in vivo* activity screenings, the plasmids were transformed into *E. coli* strains, either capable of *de novo* synthesis of sialic acid or in a strain encoding for a sialic acid importer (e.g. *E. coli nanT*) as well as a CMP-sialic acid synthetase (e.g. *C. jejuni neuA*). For *in vitro* assays an *E. coli* BL21(DE3) wild type or a *lacZ*-lacking variant thereof was used.

The activities of the screened enzymes were determined by product identification using thin layer chromatography and/or mass spectrometry analysis.

Samples analysed by thin layer chromatography (TLC) were applied on Silica Gel 60 F₂₅₄ (Merck KGaA, Darmstadt, Germany). A mixture of butanol:acetone:acetic acid:H₂O (35/35/7/23 (v/v/v/v)) was used as mobile phase. For detection of the separated substances, the TLC plate was soaked with thymol reagent (0.5 g thymol solved in 95 ml ethanol, 5 ml sulfuric acid added) and heated.

Mass spectrometry analysis was performed by MRM (multiple reaction monitoring) using a LC Triple-Quadrupole MS detection system. Precursor ions are selected and analyzed in quadrupole 1, fragmentation takes place in the collision cell using argon as CID gas, selection of fragment ions is performed in quadrupole 3. Chromatographic separation of carbohydrates was performed on a XBridge Amide HPLC column (3.5 µm, 2.1 x 50 mm (Waters, USA) with a XBridge Amide guard cartridge (3.5 µm, 2.1 x 10 mm) (Waters, USA). Column oven temperature of the HPLC system was 50°C. The mobile phase was composed of acetonitrile:H₂O with 10 mM ammonium acetate. A 1 µl sample was injected into the instrument; the run was performed for 3.60 min with a flow rate of 400 µl/min. Carbohydrates were analyzed by MRM in ESI positive ionization mode. Lactose forms an ion of m/z 341.00 [M-H]. The precursor ion of lactose was further fragmented in the collision cell into the fragment ions m/z 179.15, m/z 161.15 and m/z 101.05.

**Table 1: Enzymes with proven α-2,6-sialyltransferase activity (GT: glycosyltransferase; AA: amino acid; NA: nucleic acid)**

| Organism | Acc. No. | GT family |
|---|---|---|
| *Photobacterium sp.* | BAF92026 | GT80 |
| *Photobacterium damselae* | BAA25316 | GT80 |
| *Photobacterium leiognathi* | BAI49484 | GT80 |
| *Photobacterium leiognathi* | BAF91416 | GT80 |
| *Streptococcus suis* | AGL48117 | GT52 |
| *Streptococcus suis* | EEF64774 | GT52 |
| *Photobacterium phosphoreum* | CDN97322 | GT80 |
| *Alistipes timonensis* | WP_026020701 | GT80 |
| *Haemophilus parasuis* | KEZ22922 | GT52 |
| *Acinetobacter pittii* | EOQ74854 | GT52 |
| *Porphyromonas sp.* | KGO00880 | GT52 |
| *Photobacterium damselae* | WP_005298232 | GT80 |
| *Pasteurella multocida* | EGP04633 | GT80 |
| *Actinobacillus ureae* | WP_005624339 | GT52 |
| *Actinobacillus equuli* | WP_039196193 | GT52 |
| *Acinetobacter calcoaceticus* | WP_017391462 | GT52 |
| *Acinetobacter baumannii* | WP_032039596 | GT52 |
| *Acinetobacter oleivorans* | WP_042898369 | GT52 |
| *Acinetobacter baumannii* | ELX04805 | GT52 |
| *Acinetobacter gyllenbergii* | WP_032865812 | GT52 |
| *Shigella boydii* | ACD37071 | GT52 |
| *Kingella kingae* | WP_026036248 | GT52 |
| *Alysiella crassa* | WP_034294366 | GT52 |
| *Alistipes sp.* | EFR56292 | GT80 |
| *Streptococcus suis* | WP_024397787 | GT52 |
| *Fusobacterium mortiferum* | EEO36694 | GT52 |
| *Porphyromonas catoniae* | WP_005467220 | GT52 |

Sialyllactose forms an ion of m/z 656.2 [M+Na]. The precursor ion of sialyllactose was further fragmented in the collision cell into the fragment ions m/z 612.15, m/z 365.15 and m/z 314.15. Disialyllactose forms an ion of m/z 942.4 [M+H+NH₃]. The precursor ion of disialyllactose was further fragmented in the collision cell into the fragment ions m/z 292.30, m/z 274.30 and m/z 634.05. Collision energy, Q1 and Q3 Pre Bias were optimized for each analyte individually.

For all enzymes listed in Table 1, the formation of 6'-SL could be verified when expressed and/or assayed in the presence of lactose.

### Example 2: Fermentative production of 6'-SL using an engineered E. coli strain encoding for the α-2,6-sialyltransferase plsT6 of Photobacterium leiognathi

A 6'-sialyllactose fed-batch fermentation was conducted employing a recombinant 6'-sialyllactose synthesizing *E. coli* strain (*E. coli* BL21(DE3) Δ*lacZ*), containing a genomic integration of a α-2,6-sialyltransferase gene from *Photobacterium leiognathi* (acc. No. BAI49484). To enable/enhance the biosynthesis of CMP-sialic acid, genes encoding the glucosamine-6-phosphate synthase GlmS from E. *coli,* the

*N*-acetylglucosamine-2-epimerase Slr1975 from *Synechocystis sp.,* the gluco samine 6-phosphat N-acetyltransferase Gna1 from *Saccharomyces cerevisiae,* the phosphoenolpyruvat synthase PpsA from *E*. *coli,* the *N*-acetylneuraminate synthase NeuB and the CMP-sialic acid synthetase NeuA, the latter both from *Campylobacter jejuni,* were chromosomally integrated into the *E. coli* BL21(DE3) host. Furthermore, the gene encoding the lactose permease LacY from *E. coli,* and the genes *cscB* (sucrose permease), *cscK* (fructokinase), *cscA* (sucrose hydrolase), and *cscR* (transcriptional regulator) from *E. coli* W were integrated into the BL21 genome. Transcription of integrated genes is initiated from constitutive promotors, either the tetracycline promotor Ptet or the PT5 promotor. A functional *gal*-operon, consisting of the genes *galE* (UDP-glucose-4-epimerase), *galT* (galactose-1-phosphate uridylyltransferase), *galK* (galactokinase), and *galM* (galactose-1-epimerase) was transferred from *E. coli* K12 to the genome of the BL21 strain.

To prevent degradation of *N*-acetylglucosamine 6-phosphate genes coding for the N-acetylglucosamine-6-phosphate deacetylase (NagA), the glucosamine-6-phosphate deaminase (NagB), and the *N*-acetylglucosamine specific PTS protein IIABC (NagE) were deleted from the chromosome. Additionally, the operon *manXYZ,* encoding a sugar transporter of the *E*. *coli* PTS system for mannose, glucose, glucosamine and *N*-acetylglucosamine was deleted, as well as the genes *nanA, nanK, nanE,* and *nanT,* encoding the *N*-acetylneuraminate lyase, the *N*-acetylmannosamine kinase, the *N*-acetylmannosamine-6-phosphate epimerase, and the sialic acid transporter, respectively. The gene encoding the *N*-acetylgalactosamine-6-phosphate deacetylase (AgaA) was also deleted.

For the fermentative production of 6'-sialyllactose, the strain was grown in a defined mineral salts medium, comprising 7 g l⁻¹ NH₄H₂PO₄, 7 g l⁻¹ K₂HPO₄, 2 g l⁻¹ KOH, 0.3g l⁻¹ citric acid, 5 g l⁻¹ NH₄Cl, 1 ml l⁻¹ antifoam (Struktol J673, Schill + Seilacher), 0.1 mM CaCl₂, 8 mM MgSO₄, trace-elements (0.101 g l⁻¹ nitrilotriacetic acid, pH 6.5, 0.056 g l⁻¹ ammonium ferric citrate, 0.01 g l⁻¹ MnCl₂ x 4 H₂O, 0.002 g l⁻¹ CoCl₂ x 6 H₂O, 0.001g l⁻¹ CuCl₂ x 2 H₂O, 0.002 g l⁻¹ boric acid, 0.009 g l⁻¹ ZnSO₄ x 7 H₂O, 0.001 g l⁻¹ Na₂MoO₄ x 2 H₂O, 0.002 g l⁻¹ Na₂SeO₃, 0.002 g l⁻¹ NiSO₄ x 6 H₂O) and 2 % sucrose as carbon source.

The sucrose feed (500 g l⁻¹) was supplemented with 8 mM MgSO₄ , 0.1 mM CaCl₂, trace elements, and 5 g l ¹ NH₄Cl. For 6'-sialyllactose formation, a lactose feed of 216 g l⁻¹ was employed. The pH was controlled by using ammonia solution (25 % v/v). Fed batch fermentation was conducted at 30°C under constant aeration and agitation for 72 hours by applying a sucrose feeding rate of 5.5 - 7 mL L⁻¹ h⁻¹, referring to the starting volume. Lactose that was not converted into 6'-sialyllactose at the end of the production process was degraded by addition of β-galactosidase and monosaccharides from hydrolysis of lactose were metabolized by the production strain. Substantial amounts of 6'-SL were excreted by the bacterial cells into the culture supernatant. However, in addition, a disialyllactose was formed (Figure 2), which could be verified by mass spectrometry analysis as well as thin layer chromatography. The ratio of 6'6-Di-SL to 6'-SL in the culture broth, determined by HPLC, was approximately 8 %.

High-performance liquid chromatography (HPLC) analysis, using a refractive index detector (RID-10A) (Shimadzu, Germany) and a Waters XBridge Amide Column 3.5µm (250 x 4.6mm) (Eschborn, Germany) connected to an HPLC system (Shimadzu, Germany), was conducted to estimate the ratio of 6'6-disialyllactose to 6'-sialyllactose within the fermentation broth. Elution was performed isocratically with 71 % (v/v) ACN in ddH₂O (adjusted to pH 4.2 with acetic acid) at 35 °C and at a flow rate of 1.4 mlmin⁻¹. HPLC samples were sterile filtered (0.22 µm pore size) and heated to 80 °C for 5 minutes prior to injection. 10 µl of the samples were applied to the column. The retention times of 6'-SL and 6'6-Di-SL were determined by applying purified standards. The ratio of 6'6-Di-SL to 6'-SL can be determined by building the quotient of the AUC (area under the curve) of the corresponding peaks in the chromatogram.

### Example 3: Novel α-2,6-sialyltransferases for the production of 6'-sialyllactose

Sialyltransferases originating from *Streptococcus suis* demonstrated a good sialyltransferase activity when using galactosides (e.g. galactose or lactose) as acceptor substrates, thus, revealing an alternative to α-2,6-sialyltransferases originating from marine bacteria. No annotated/putative *S. suis* sialyltransferase has ever before been characterized as a lactose accepting enzyme, thus, no *S.* suis sialyltransferase has been applied for 6'-SL production so far.

For an in-depth characterization, *in vitro* enzyme assays were conducted. Therefore, *Escherichia coli* BL21(DE3) harbouring plasmids encoding the sialyltransferases were grown at 30 °C in 100 ml shake flasks filled with 20 ml of 2YT medium supplemented with ampicillin 100 µg ml⁻¹. When the cultures reached an OD₆₀₀ of 0.1 to 0.3, gene expression was induced by addition of 0.3 mM IPTG and the incubation was continued for 12 to 16 hours. Cells were harvested by centrifugation and mechanically disrupted in a defined volume of 50 mM Tris-HCl pH7.5 using glass beads. The protein extract was kept on ice until the assay started.

**Table 2: Summary of candidate α-2,6-sialyltransferases tested for their ability to accept different carbohydrates for sialylation during in vivo and/or in vitro assays. The activity of each sialyltransferase was semi-quantitatively compared, separately for each acceptor substrate, to Pst-6.**

| Protein name | Organism | Acc. No. | Identity with Pst-6 [%] | Sialyltransferase activity on different acceptor substrates | | |
|---|---|---|---|---|---|---|
| | | | | Lactose | 6'-SL | 3'-SL |
| Pst-6 | *Photobacterium sp.* | BAF92026 | - | +++ | + | + |
| St0160 | *Photobacterium damselae* | BAA25316 | 39,6 | +++ | + | + |
| PIsT6 | *Photobacterium leiognathi* | BAI49484 | 54,2 | +++ | + | + |
| PIsT6 | *Photobacterium leiognathi* | BAF91416 | 52,3 | +++ | + | + |
| Cpa16Q | *Streptococcus suis* | AGL48117 | 12,2 | ++ | - | - |
| Cps1O | *Streptococcus suis* | EEF64774 | 13,5 | ++ | - | - |

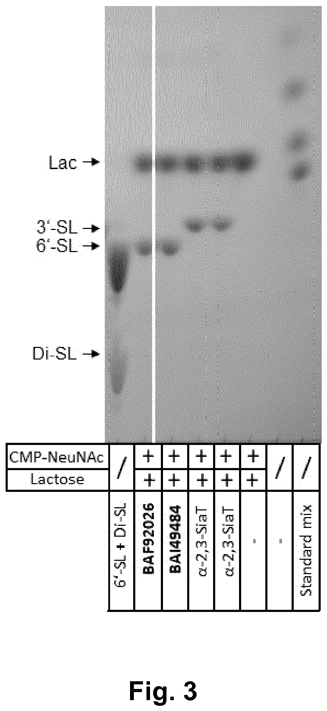

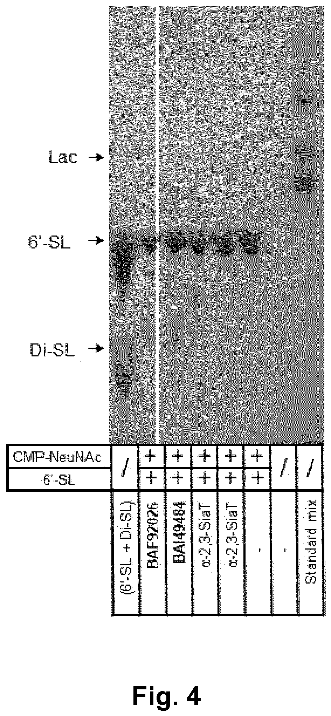

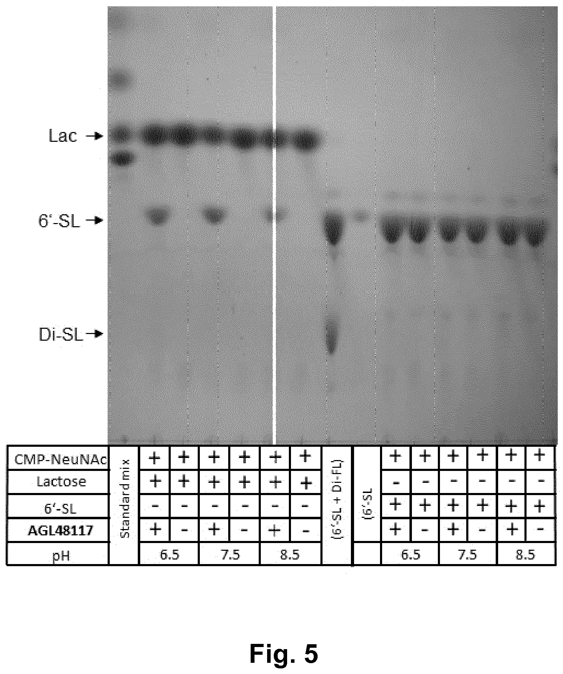

For determining their sialyltransferase properties, the *in vitro* assays were carried out in a total volume of 25 µl including 50 mM Tris-HCl pH7.5, 5 mM MgCl₂, 10 mM CMP-Neu5Ac and varying concentrations of acceptor substrates such as lactose, 3'-SL or 6'-SL. The assay started with the addition of 3 µl protein extract and continued for up to 16 hours. Product formation was determined by thin layer chromatography and was verified by mass spectrometry. Exemplary results of these *in vitro* assays are depicted in FIG. 3, 4 and 5. Whereas *Photobacterium* α-2,6-sialyltransferases are capable to convert lactose as well as 6'-SL to 6'-SL and Di-SL, respectively, the *Streptococcus suis* enzyme solely demonstrates detectable α-2,6-sialyltransferase activity when lactose is provided as acceptor substrate. The summarized results can be found in Table 2. Surprisingly, neither of the S. *suis* sialyltransferases was capable of disialyllactose formation.

For the determination of a sialidase/trans-sialidase activity, the *in vitro* assays were carried out in a total volume of 25 µl including 50 mM Tris-HCl pH7.5, 5 mM MgCl₂ and varying concentrations of 3'-SL or 6'-SL. The assay started with the addition of 3 µl protein extract and continued for up to 16 hours. Lactose and/or disialyllactose formation was determined by thin layer chromatography and/or mass spectrometry. In contrast to the sialyltransferases originating from marine bacteria, neither of the S. *suis* sialyltransferases was capable of converting 3'-SL and/or 6'-SL to lactose and/or disialyllactose (Table 3).

**Table 3: Summary of candidate α-2,6-sialyltransferases tested for their sialidase/trans-sialidase activity during in vitro assays. The activity of each enzyme was semi-quantitatively compared to Pst-6.**

| Protein name | Organism | Acc. No. | Sialidase/Trans-sialidase activity |
|---|---|---|---|
| Pst-6 | *Photobacterium sp.* | BAF92026 | + |
| St0160 | *Photobacterium damselae* | BAA25316 | + |
| PIsT6 | *Photobacterium leiognathi* | BAI49484 | + |
| PlsT6 | *Photobacterium leiognathi* | BAF91416 | + |
| Cpa16Q | *Streptococcus suis* | AGL48117 | - |
| Cps1O | *Streptococcus suis* | EEF64774 | - |

### Example 4: Fermentative production of 6'-SL using an engineered E. coli strain encoding for the α-2,6-sialyltransferase cps16Q of Streptococcus suis

An *E*. *coli* strain, metabolically engineered as described in example 2 but containing a genomic integration of the α-2,6-sialyltransferase gene from *Streptococcus suis* (acc. No. AGL48117), was applied to a fed-batch fermentation. The cultivation conditions are described in example 2. After discontinuation of the cultivation, substantial amounts of 6'-SL, comparable to the process described in example 2, were detectable in the culture supernatant but no disialyllactose.

## Claims

1. A genetically engineered cell for the production of 6'-sialyllactose, wherein said cell has been genetically engineered to possess a lactose-accepting α-2,6-sialyltransferase which comprises an amino acid sequence that is at least 98 % identical to one of the amino acid sequences as represented by SEQ ID No. 1 and SEQ ID No. 2, wherein the α-2,6-sialyltransferase is not able to catalyze formation of disialyllactose.

2. The genetically engineered cell according to claim 1, wherein the cell is selected from the group consisting of prokaryotic cells and eukaryotic cells, preferably from the group consisting of yeast cells, bacterial cells, archaebacterial cells, algae cells, and fungal cells.

3. The genetically engineered cell according to claim 1 or 2, wherein the α-2,6-sialyltransferase comprises an amino acid sequence as represented by SEQ ID No. 1 or SEQ ID No. 2.

4. The genetically engineered cell according to any one of claims 1 to 3, wherein the cell contains a nucleic acid molecule which comprises a nucleotide sequence which encodes the lactose-accepting α-2,6-sialyltransferase which comprises an amino acid sequence that is at least 98 % identical to one of the amino acid sequences as represented by SEQ ID No.1 and SEQ ID No. 2, wherein the α-2,6-sialyltransferase is not able to catalyze formation of disialyllactose.

5. The genetically engineered cell according to claim 4, wherein the nucleotide sequence is selected from the group consisting of nucleotide sequences encoding a polypeptide as represented by any one of SEQ ID No's: 1 and 2.

6. A method for producing 6'-sialyllactose in a cell, the method comprises
- providing a genetically engineered cell for the production of 6'-sialyllactose, wherein said cell has been genetically engineered to possess a lactose-accepting α-2,6-sialyltransferase which comprises an amino acid sequence that is at least to 98 % identical to one of the amino acid sequences as represented by SEQ ID No. 1 and SEQ ID No. 2, wherein the α-2,6-sialyltransferase is not able to catalyze formation of disialyllactose;
- cultivating said cell in a culture medium and under conditions permissive for the production of 6'-sialyllactose; and
- optionally recovering said 6'-sialyllactose.

7. The method according to claim 6, wherein the culture medium contains at least one carbon source selected from the group consisting of glucose, fructose, sucrose, and glycerol.

8. The method according to claim 6 or 7, wherein the culture medium contains at least one compound selected from the group consisting of lactose, galactose and sialic acid.

9. The method according to any one of claims 6 to 8, wherein the 6'-sialyllactose is recovered from the culture medium and/or the bacterial cell.

## Patentansprüche

1. Gentechnisch veränderte Zelle zur Herstellung von 6'-Sialyllactose, wobei die Zelle gentechnisch verändert wurde, sodass sie eine Lactose-akzeptierende α-2,6-Sialyltransferase besitzt, die eine Aminosäuresequenz umfasst, die zu mindestens 98 % identisch mit einer der Aminosäuresequenzen ist, wie durch SEQ ID No. 1 und SEQ ID No. 2 dargestellt, wobei die α-2,6-Sialyltransferase nicht in der Lage ist, die Bildung von Disialyllactose zu katalysieren.

2. Gentechnisch veränderte Zelle nach Anspruch 1, wobei die Zelle aus der Gruppe ausgewählt ist, die aus prokaryotischen Zellen und eukaryotischen Zellen besteht, vorzugsweise aus der Gruppe, die aus Hefezellen, Bakterienzellen, Archaebakterienzellen, Algenzellen und Pilzzellen besteht.

3. Gentechnisch veränderte Zelle nach Anspruch 1 oder 2, wobei die α-2,6-Sialyltransferase eine Aminosäuresequenz umfasst, wie durch SEQ ID No. 1 oder SEQ ID No. 2 dargestellt.

4. Gentechnisch veränderte Zelle nach einem der Ansprüche 1 bis 3, wobei die Zelle ein Nukleinsäuremolekül enthält, das eine Nukleotidsequenz umfasst, die für die Lactose-akzeptierende α-2,6-Sialyltransferase kodiert, die eine Aminosäuresequenz umfasst, die zu mindestens 98 % identisch mit einer der Aminosäuresequenzen ist, wie durch SEQ ID No. 1 und SEQ ID No. 2 dargestellt, wobei die α-2,6-Sialyltransferase nicht in der Lage ist, die Bildung von Disialyllactose zu katalysieren.

5. Gentechnisch veränderte Zelle nach Anspruch 4, wobei die Nukleotidsequenz ausgewählt ist aus der Gruppe bestehend aus Nukleotidsequenzen, die für ein Polypeptid kodieren, wie dargestellt durch eine der SEQ ID No. 1 und 2.

6. Verfahren zum Herstellen von 6'-Sialyllactose in einer Zelle, wobei das Verfahren Folgendes umfasst
- Bereitstellen einer gentechnisch veränderten Zelle zur Herstellung von 6'-Sialyllactose, wobei die Zelle gentechnisch verändert wurde, sodass sie eine Lactose-akzeptierende α-2,6-Sialyltransferase besitzt, die eine Aminosäuresequenz umfasst, die zu mindestens bis 98 % identisch mit einer der Aminosäuresequenzen ist, wie durch SEQ ID No. 1 und SEQ ID No. 2 dargestellt, wobei die α-2,6-Sialyltransferase nicht in der Lage ist, die Bildung von Disialyllactose zu katalysieren;
- Kultivieren der Zelle in einem Kulturmedium und unter Bedingungen, die die Herstellung von 6'-Sialyllactose erlauben; und
- optional Gewinnen der 6'-Sialyllactose.

7. Verfahren nach Anspruch 6, wobei das Kulturmedium mindestens eine Kohlenstoffquelle enthält, die aus der Gruppe ausgewählt ist, die aus Glucose, Fructose, Sucrose und Glycerin besteht.

8. Verfahren nach Anspruch 6 oder 7, wobei das Kulturmedium mindestens eine Verbindung enthält, die ausgewählt ist aus der Gruppe bestehend aus Lactose, Galactose und Sialinsäure.

9. Verfahren nach einem der Ansprüche 6 bis 8, wobei die 6'-Sialyllactose aus dem Kulturmedium und/oder der Bakterienzelle gewonnen wird.

## Revendications

1. Cellule génétiquement modifiée pour la production de 6'-sialyllactose, dans laquelle ladite cellule a été génétiquement modifiée pour posséder une α-2,6-sialyltransférase acceptant le lactose qui comprend une séquence d'acides aminés qui est identique à au moins 98 % à l'une des séquences d'acides aminés telles que représentées par SEQ ID N° 1 et SEQ ID N° 2, dans laquelle 1'α-2,6-sialyltransférase n'est pas capable de catalyser la formation de disialyllactose.

2. Cellule génétiquement modifiée selon la revendication 1, dans laquelle la cellule est choisie dans le groupe constitué par des cellules procaryotes et des cellules eucaryotes, de préférence dans le groupe constitué par des cellules de levure, des cellules bactériennes, des cellules archéobactériennes, des cellules d'algues et des cellules fongiques.

3. Cellule génétiquement modifiée selon l'une des revendications 1 ou 2, dans laquelle 1'α-2,6-sialyltransférase comprend une séquence d'acides aminés telle que représentée par SEQ ID N° 1 ou SEQ ID N° 2.

4. Cellule génétiquement modifiée selon l'une quelconque des revendications 1 à 3, dans laquelle la cellule contient une molécule d'acide nucléique qui comprend une séquence nucléotidique qui code pour 1'α-2,6-sialyltransférase acceptant le lactose qui comprend une séquence d'acides aminés qui est identique à au moins 98 % à l'une des séquences d'acides aminés telles que représentées par SEQ ID N° 1 et SEQ ID N° 2, dans laquelle 1'α-2,6-sialyltransférase n'est pas capable de catalyser la formation de disialyllactose.

5. Cellule génétiquement modifiée selon la revendication 4, dans laquelle la séquence nucléotidique est choisie dans le groupe constitué de séquences nucléotidiques codant pour un polypeptide tel que représenté par l'une quelconque des SEQ ID N° : 1 et 2.

6. Procédé permettant la production de 6'-sialyllactose dans une cellule, le procédé comprend
- la fourniture d'une cellule génétiquement modifiée pour la production de 6'-sialyllactose, dans lequel ladite cellule a été génétiquement modifiée pour posséder une α-2,6-sialyltransférase acceptant le lactose qui comprend une séquence d'acides aminés qui est identique à au moins 98 % à l'une des séquences d'acides aminés telles que représentées par SEQ ID N° 1 et SEQ ID N° 2, dans lequel 1'α-2,6-sialyltransférase n'est pas capable de catalyser la formation de disialyllactose ;
- la culture de ladite cellule dans un milieu de culture et dans des conditions permettant la production de 6'-sialyllactose ; et
- éventuellement la récupération dudit 6'-sialyllactose.

7. Procédé selon la revendication 6, dans lequel le milieu de culture contient au moins une source de carbone choisie dans le groupe constitué de glucose, de fructose, de saccharose et de glycérol.

8. Procédé selon l'une des revendications 6 ou 7, dans lequel le milieu de culture contient au moins un composé choisi dans le groupe constitué par le lactose, le galactose et l'acide sialique.

9. Procédé selon l'une quelconque des revendications 6 à 8, dans lequel le 6'-sialyllactose est récupéré à partir du milieu de culture et/ou de la cellule bactérienne.
